# FASCICULE DE BREVET EUROPEEN

(11) **EP 2 694 054 B1**
(45) Date de publication et mention de la délivrance du brevet: **13.06.2018**
(21) Numéro de dépôt: 12720245.5
(22) Date de dépôt: 05.04.2012
(51) Int. Cl.: A61K 31/185, A61K 31/255, A61K 31/315, A61P 35/00

(54) **N-ACETYL-TAURINATE DE ZINC POUR LE TRAITEMENT DU CANCER DE LA PROSTATE**
ZINK N-ACETYL-TAURINAT ZUR BEHANDLUNG VON PROSTATAKREBS
ZINC N-ACETYL-TAURINATE FOR THE TREATMENT OF PROSTATE CANCER

(30) Priorité: 08.04.2011 FR 1153055
(43) Date de publication de la demande: 12.02.2014
(73) Titulaire: Tri-Inov, 92200 Neuilly sur Seine (FR)
(72) Inventeur: DURLACH, Jean Pierre, F-92200 Neuilly Sur Seine (FR)
(74) Mandataire: Mendelsohn, Isabelle M. N.
(86) Numéro de dépôt international: PCT/FR2012/050739
(87) Numéro de publication internationale: WO 2012/136937

(56) Documents cités:
- WO-A1-02/074294
- WO-A2-2007/109290
- FR-A1- 2 927 809
- US-A- 4 946 688
- US-A- 5 672 592
- US-A1- 2010 183 740
- WEI HUA ET AL: "Differential expression of metallothioneins (MTs) 1, 2, and 3 in response to zinc treatment in human prostate normal and malignant cells and tissues", MOLECULAR CANCER, BIOMED CENTRAL, LONDON, GB, vol. 7, no. 1, 21 janvier 2008 (2008-01-21), page 7, XP021036989, ISSN: 1476-4598

## Description

La présente invention a pour objet une nouvelle utilisation du N-acétyl-taurinate de zinc. WEI HUA ET AL: "Differential expression of metallothioneins (MTs) 1, 2, and 3 in response to zinc treatment in human prostate normal and malignant cells and tissues", MOLECULAR CANCER, BIOMED CENTRAL, LONDON, GB, vol. 7, no. 1, 21 janvier 2008 (2008-01-21), page 7,ISSN: 1476-4598 suggère l'efficacité du zinc dans la prévention et le traitement du cancer de prostate. Le N-acétyl-taurinate de zinc appartient à la famille des dérivés de, la taurine à activité neuro-musculaire renforcée, qui sont décrits dans le brevet FR 2 384 751. Dans la demande WO 2009/112758 on a décrit l'utilisation du N-acétyl-taurinate de zinc pour prévenir et/ou traiter les maladies avec accumulation de lipofuscine due au vieillissement ou à un stress oxydatif.

On a maintenant trouvé de façon surprenante que le N-acétyl-taurinate de zinc (ATA-Zn) peut être utilisé comme agent anticancéreux pour le traitement du cancer de la prostate.

Ainsi, la présente invention a pour objet le N-acétyl-taurinate de zinc de formule :

[CH₃-CO-NH-CH₂-CH₂-SO₃]⁻₂Zn²⁺

pour traiter le cancer de la prostate.

L'invention a également pour objet N-acétyl-taurinate de zinc pour son utilisation dans le traitement du cancer de la prostate. Le N-acétyl-taurinate de zinc est préparé par action de l'anhydride acétique sur la taurine en présence d'acétate de zinc selon un procédé analogue à celui décrit pour la préparation du N-acétyl-taurinate de sodium par M. TERAKOA (Hoppe-Seyler Zeitschrift für Physiologische Chemie, 145, 242 (1925)).

De préférence, on utilise le N-acétyl-taurinate de zinc dihydraté.

Pour l'administration aux patients atteints du cancer de la prostate, le N-acétyl-taurinate de zinc est avantageugement mélangé en tant que principe actif avec un excipient pharmaceutiquement acceptable couramment utilisé pour la préparation de compositions pharmaceutiques administrables par voie orale, parentérale ou locale.

L'activité anticancéreuse de ATA-Zn a été démontrée en utilisant des cellules cancéreuses et des cellules normales de la prostate.

Les tests réalisés ont montré que l'ATA-Zn a un effet anti-prolifératif sur des cellules cancéreuses de la prostate alors qu'il n'a aucun effet sur des cellules normales de la prostate.

De plus, on a montré que cet effet antiprolifératif était concomittant avec un arrêt du cycle cellulaire via la répression de l'ARNm de la cycline-D1 et avec une augmentation de l'apoptose via la régulation transcriptionnelle positive de l'indice d'apoptose Bax/Bcl-2.

Par ailleurs, on a montré que l'ATA-Zn était atoxique à des concentrations inférieures à 10⁻³M.

L'invention va être maintenant décrite plus en détail par la préparation et les tests ci-après.

### PREPARATION

On a mélangé 20,25 g de taurine et 17,5 g d'acétate de zinc pur sec et on a ajouté 50 g d'eau pure.

On a chauffé la suspension résultante à une température comprise entre 65 et 75°C et on a ajouté à cette suspension, 45 g d'anhydride acétique puis on a chauffé à 100-105°C. On a ensuite ajouté 100 ml d'éthanol anhydre au mélange réactionnel résultant à une température comprise entre 70 et 75°C.

On a finalement obtenu 30 ± 3 g du produit attendu sous la forme d'une poudre blanche soluble dans l'eau et peu soluble dans l'éthanol (rendement pondéral : 36,25 %).

### Analyse (en pourcentages)

| **Analyse** | **Calculé** | **Trouvé** |
|---|---|---|
| C | 24,16 | 22,75 |
| H | 4,05 | 4,60 |
| N | 7,04 | 5,73 |
| Zn* | 16,4 | 16,7 |

| | | |
|---|---|---|
| * *dosage de Zn par EDTA* | | |

### TESTS IN VITRO

### a) Matériels et méthodes

Dans les tests ci-après, on a utilisé les cultures cellulaires et millieux de culture ci-après:

### Cultures cellulaires et milieux cellulaires

On a utilisé la lignée de cellules cancéreuses de la prostate LNCaP, déposée à l'ATCC sous le n°CRL-1740.

Le milieu de culture des cellules était le milieu de croissance complet RPMI 1640 (ATCC n°30-2001), contenant de la L-glutamine 2 mM, de l'HEPES 10 mM et du pyruvate de sodium 1,0 mM, auquel on a ajouté :
1) du carbonate de sodium et du glucose en quantités suffisantes pour obtenir une concentration de 20mM en carbonate de sodium et de 25mM en glucose,
2) 90% en volume d'un mélange d'antibiotiques (10 µg/ml de gentamycine, 100 UI de pénicilline, 100 µg/ml de streptomycine), et
3) 10% en volume de sérum de veau foetal.

Des cellules épithéliales normales de la prostate (PrEC) et le milieu de croissance PrEC commercialisés par la Société Cambrex Bioscience (Walkersville, MD), ont été utilisés à titre de comparaison.

### Traitement des cellules

Pour les études de dose-dépendance, les cellules (confluentes à 50 %) ont été traitées avec de l'ATA-Zn aux concentrations de 10⁻³, 10⁻⁴, 10⁻⁵, 10⁻⁶, 10⁻⁷, 10⁻⁸, et 10⁻⁹M, pendant 24 heures, 48 heures, et 72 heures dans leur milieu de croissance respectif. Au bout de 48 h de traitement, les cellules ont été récoltées et comptées dans le culot. Parallèlement, les lysats cellulaires ont été préparés pour extraire l'ARNm pour la RT-Q-PCR et le Western blot.

### Numération cellulaire

Afin d'évaluer le nombre de cellules, les culots cellulaires ont été mélangés avec 1 ml de leur milieu respectif et la viabilité cellulaire ainsi que le nombre de cellules ont été évalués par un test d'exclusion au bleu de Trypan.

### RT-O-PCR (réaction de polymérisation en chaîne et en temps réel)

L'ARNₘ a été extrait des cellules en culture en utilisant le kit intitulé « SV Total RNA isolation » de la Société Promega et en suivant le mode opératoire ci-après : Les cellules sont récoltées par aspiration du milieu de culture et lavées au PBS (3 fois x 4 ml) à température ambiante pendant 5 min.

On ajoute ensuite 175 µl de tampon de lyse SV RNA aux cellules lavées, puis on disperse la pelotte d'ARN et on mélange bien à l'aide d'un vortex.

On répète l'opération plusieurs fois et on met le lysat cellulaire obtenu dans des tubes de 1,5 ml. Jusqu'à 1x10⁶ cellules sont facilement lysées dans 175 µl du tampon de lyse utilisé.

On ajoute ensuite 350 µl de tampon de dilution SV RNA au 175 µl de lysat. On mélange par retournement des tubes 3 à 4 fois.

Les tubes sont ensuite mis au bain-marie à 70°C pendant 3 min maximum.

Ensuite, on centrifuge pendant 10 min à 13000 g et à température ambiante. Le surnageant clair obtenu est récupéré dans des microtubes stériles de 1,5 ml. On ajoute ensuite 200 µl d'éthanol 95% et on mélange en pipetant 3 à 4 fois.

On ajoute le mélange alcoolique obtenu ci-dessus sur la colonne de séparation fournie avec le kit, on centrifuge à 13000 g pendant 1 min et on ajoute 600 µl de solution de lavage et on centrifuge à nouveau à 13000 g pendant 1 min.

On prépare la solution de DNase en mélange 40 µl de tampon d'extraction, 5 µl de MnCl₂ et 5 µl de DNase.

On ajoute 50 µl de la solution DNase sur la membrane de la colonne de séparation et on laisse incuber pendant 15 min à température ambiante.

On ajoute ensuite 200 µl de solution d'arrêt SV DNase, on centrifuge pendant 1 min à 13000 g, on ajoute 600 µl de solution de lavage, on centrifuge une nouvelle fois à 13000 g pendant 1 min, puis on ajoute 250 µl de solution de lavage et on centrifuge à 13000 g pendant 2 min. On place les colonnes dans les tubes d'élution stériles fournis dans le kit, on ajoute 100 µl d'eau fournie avec le kit, et on centrifuge pendant 1 min à 13000 g pour éluer l'ARNm. L'ARNm ainsi récupéré est stocké à -80°C.

On contrôle la qualité de l'ARNm obtenu et on détermine la concentration en ARNm dans l'échantillon obtenu selon les modes opératoires classiques bien connus de l'homme du métier.

Ensuite, une transcription inverse de l'ARN a été réalisée en utilisant le test prêt à l'emploi (kit dénommé en langue anglaise « Ready To Go, You Prime First Strand Beads » disponible commercialement auprès de Amersham Biosciences, Piscataway, États-Unis) conformément aux instructions du fournisseur à partir de 3 µg d'ARN.

Le test « LightCycler-Fast Start DNA Master SYBR Green I » disponible commercialement auprès de Roche Diagnostics, Meylan, France, a été utilisé pour quantifier l'expression génique par une réaction de polymérisation en chaîne (PCR) en temps réel. La PCR (programme : 95°C, 30 secondes ; 40 cycles : 95°C, 5 secondes ; 60°C, 35 secondes) a été réalisée à l'aide d'un appareil « Mastercycler ep realplex » commercialisé par la Société Eppendorf, Hambourg, Allemagne.

L'expression du gène cible a été normalisée par rapport au gène domestique GAPDH. La méthode du 2^{-ΔΔCt} a été appliquée afin de calculer l'expression génique relative.

Les amorces suivantes ont été utilisées pour la PCR :
CYCLINE D1 :
   - amorce sens : 5'-GGATGCTGGAGGTCTGCGAGGAAC 3' (SEQ ID N°1)
   - amorce antisens : 5'-GAGAGGAAGCGTGTGAGGCGGTAG-3' (SEQ ID N°2)
BAX :
   - amorce sens, 5'-TTTGCTrCAGGGTTTCATCC-3' ; (SEQ ID N°3)
   - amorce antisens, 5'-CAGTTGAAGTTGCCGTCAGA-3' ; (SEQ ID N°4)
BCL-2 :
   - amorce sens, 5'AATGCAGTGGTGCTTACGCTC-3' ; (SEQ ID N°5)
   - amorce antisens 5'-GGATAGCAGCACAGGATTGGAT-3' ; (SEQ ID N°6)
GAPDH :
   - amorce sens, 5'-GAAGGTGAAGGTCGGAGTC-3' ; (SEQ ID N°7)
   - amorce antisens 5'-GAAGATGGTGATGGGATTTC-3'. (SEQ ID N°8)

### Western blot

Les cellules ont été lysées dans le tampon pour le test de radioimmunoprécipitation ayant la composition ci-après :
Tris-HCl 50mM, pH 7,4, NaCl 150mM, Triton X-100 à 1 %, SDS à 0,1 %, NaF 50mM, Na₃VO₄ 2mM, acide okadaïque 100 nM, β-glycérophosphate 25mM, fluorure de phénylméthylsulfonyle 1mM, cocktail d'inhibiteurs de protéases de la Société Sigma contenant :
AEBSF (fluorure de 4-(2-aminoéthyl)-benzène sulfonyle) 2 mM ;
aprotinine 0,3 µM ;
bestatine 130 µM ;
EDTA 1 mM ;
E-64 14 µM ;
leupeptine 1 µM.
Ensuite, les protéines ont été séparées par SDS-PAGE et ont été transférées sur une membrane en PVDF Hybond-P (Amersham Biosciences, Pantin, France). Les membranes ont été incubées pendant 1 heure à température ambiante avec une solution de blocage constituée de poudre de lait écrémé à 5 % dans le tampon TBS-T (Tris-HCl 50mM, pH 8,0, NACl 150mM, Tween-20 à 0,1 %).

Les membranes ont ensuite été incubées avec des anticorps primaires de lapin dirigés contre la cycline-D1 (n°29785); les marqueurs Bax (n°1063.1) et BCL-2 (n°1017-1); Nrf-2 (n°2178-1) et SOD-1 (n°2018-1) tous commercialisés par la Société Epitomies sous les références indiquées ci-dessus et les anticorps anti-actine, N-terminal, A2103, commercialisés par la Société Sigma-Aldrich, (Saint Quentin Fallavier, France) dans la solution de blocage toute une nuit à 4°C.

Lesdits anticorps ont été dilués conformément aux instructions du fabricant à 1/1000. Les anticorps anti-actine ont servi à normaliser les membranes.

Les membranes ont été lavées trois fois dans le tampon TBS-T, puis ont été incubées avec des anticorps de chèvre anti-IgG de lapin conjuguées à la peroxydase du raifort (1:5000) (Santa Cruz, CA, États-Unis) dans la solution de blocage pendant 1 heure à température ambiante.

Après trois lavages, les membranes ont été développées en utilisant le système de détection amélioré de la chimioluminescence, connu sous le dénomination anglaise « ECL Prime Western Blotting Détection Reagent », commercialisé par (Amersham Biosciences (Pantin, France).

### b) Test 1 : Numérations cellulaires

### - cellules cancéreuses LNCaP

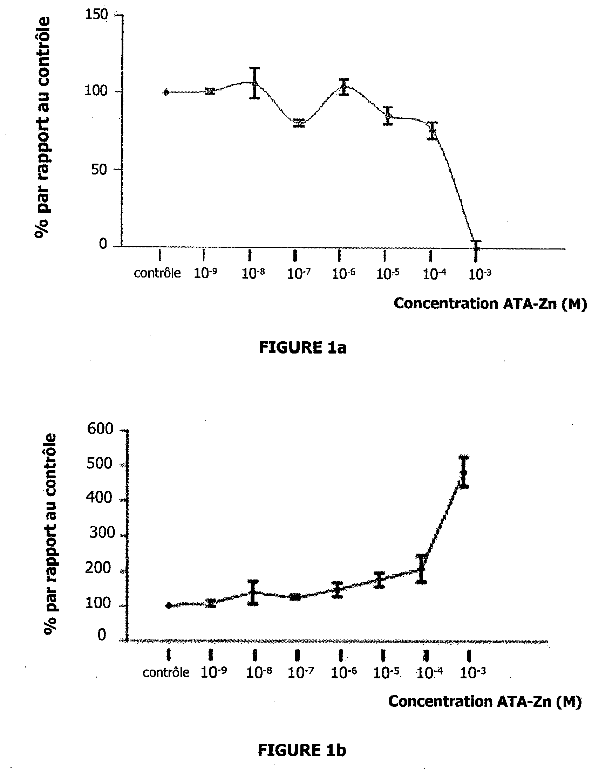

La numération des cellules viables et des cellules non viables a été réalisée après une exclusion au bleu Tryptan. Au bout de 48 heures d'incubation, on a constaté que l'ATA-Zn réduisait le nombre de cellules à chaque dose ; une réduction maximale de 25 % a été observée à la concentration la plus élevée (10⁻⁴). La mortalité évoluait dans le sens inverse avec une augmentation maximale avoisinant 50 %. La concentration de 10⁻³M était toxique. Les résultats obtenus sont reportés sur les graphes des figures 1a et 1b qui donnent le pourcentage de cellules cancéreuses par rapport au contrôle (culture cellulaire sans ATA-Zn) en fonction de la concentration en ATA-Zn. La figure la concerne les cellules viables et la figure 1b concerne les cellules non viables.

### - cellules normales PrEC

On a procédé de la même manière avec les cellules épithéliales de la prostate normale (PrEC) et on a constaté que l'ATA-Zn n'avait aucun effet sur le nombre de cellules même après 72 heures d'incubation à l'exception de la dose d'ATA-Zn de 10⁻³M qui était toxique pour les cellules, celles-ci étant complètement mortes au bout de 72 heures d'incubation avec ATA-Zn 10⁻³M.

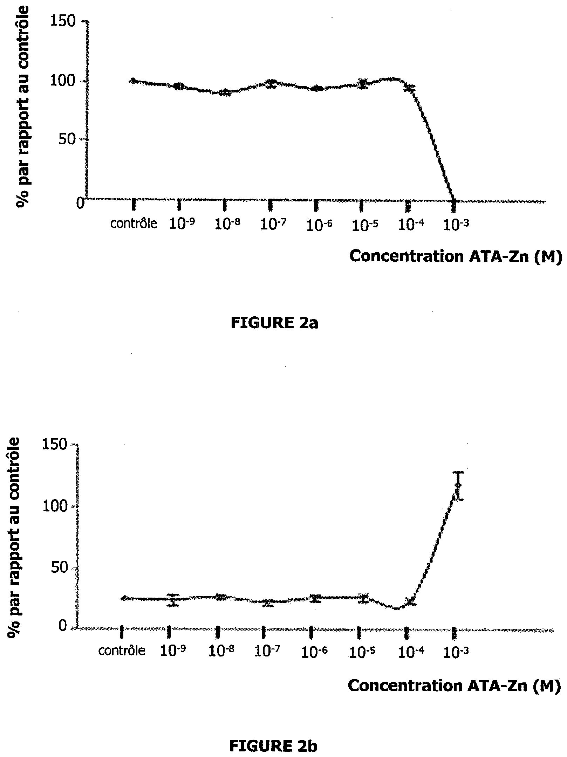

Les résultats obtenus sont reportés sur les graphes des figures 2a et 2b qui donnent le pourcentage de cellules normales par rapport au contrôle (culture cellulaire sans ATA-Zn) en fonction de la concentration en ATA-Zn. La figure 2a concerne les cellules viables et la figure 2b concerne les cellules non viables.

### c) Test 2 : Diminution de l'ARNm de cycline-D1

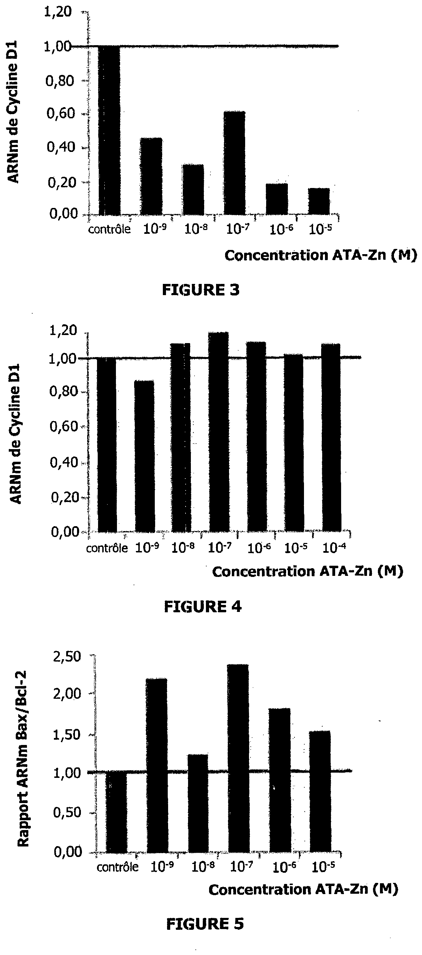

Des cellules LNCaP ont été incubées avec différentes concentrations d'ATA-Zn (10⁻⁵-10⁻⁹M) pendant 48 heures. La quantité de l'ARNm de cycline-D1 a été mesurée par RT-Q-PCR en suivant le mode opératoire décrit précédemment dans le paragraphe intitulé "Matériels et méthodes". On a constaté que ATA-Zn réduit la synthèse de l'ARNm de cycline-D1 au bout de 24 heures d'exposition comme le montrent les résultats reportés sur le graphe de la figure 3 qui donne la quantité de l'ARNm de cycline-Dl produit en fonction de la concentration d'ATA-Zn. On a répété le même test avec les cellules normales et on a constaté que l'ATA-Zn n'avait aucun effet sur la synthèse de l'ARNm de cycline-D1 comme le montre la figure 4.

### d) Test 3 : Augmentation du rapport Bax/Bcl-2

Des cellules LNCaP ont été incubées avec différentes concentrations d'ATA-Zn (10⁻⁵-10⁻⁹M) pendant 48 heures. L'évolution de la synthèse des ARNm de Bax et de Bcl-2 a été mesurée par RT-Q-PCR en suivant le mode opératoire décrit précédemment dans le paragraphe intitulé "Matériels et méthodes". On a constaté que ATA-Zn augmentait fortement le rapport Bax/Bcl-2 comme le montrent les résultats reportés sur le graphe de la figure 5 qui donne le rapport Bax/Bcl 2 en fonction de la concentration d'ATA-Zn.

On a réalisé le même test avec des cellules PrEC. Avec ces cellules normales, l'accumulation de Bax et de Bcl-2 a été impossible à déterminer car la quantité de base était trop faible pour une bonne amplification par PCR. On peut en conclure que l'apoptose est très faible pour les cellules normales PrEC dans les conditions basales et qu'elle n'est pas influencée par l'ATA-Zn.

### e) Test 4 : Influence de l'ATA-Zn sur la synthèse des protéines Nrf-2 et SOD-1

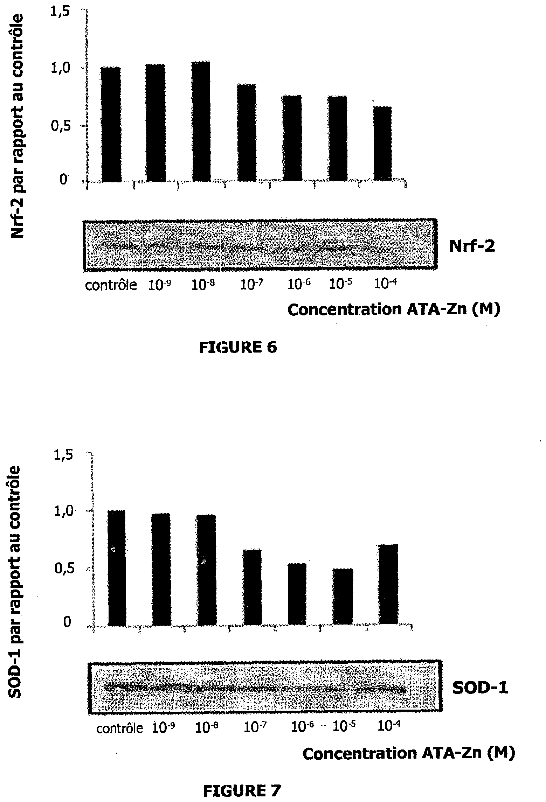

Des cellules LNCaP ont été incubées avec une gamme de concentrations de ATA-Zn pendant 48 heures. On a ensuite mesuré la quantité des protéines Nrf-2 et SOD-1 par Western blot. La quantification a été réalisée avec le logiciel « Quantity One » et les résultats ont été normalisés par rapport aux données de l'actine. Les résultats obtenus sont reportés sur la figure 6 (protéine Nrf-2) et la figure 7 (protéine de SOD-1).

Ces résultats montrent que ATA-Zn diminue le taux de la protéine SOD-1 via un mécanisme médié par un ARE (« antioxydant response element » ou élément responsable de l'anti-oxydation) puisque la protéine Nrf-2 est également réprimée. La protéine Nrf-2 est un facteur de transcription affecté par l'état redox intracellulaire. Elle régule la transcription des gènes qui possède un élément responsable de l'anti-oxydation dans leur promoteur. Ainsi, on pense que l'ATA-Zn modifie le status oxydatif des cellules cancéreuses entraînant une diminution de la croissance et/ou une augmentation de l'apoptose des cellules.

## Revendications

1. N-acétyl-taurinate de zinc de formule :
[CH₃-CO-NH-CH₂-CH₂-SO₃]⁻₂Zn²⁺
pour son utilisation dans le traitement du cancer de la prostate.

## Patentansprüche

1. Zink-N-Acetyl-Taurinat der Formel:
[CH₃-CO-NH-CH₂-CH₂-SO₃]⁻₂Zn²⁺ zur Verwendung bei der Behandlung von Prostatakrebs.

## Claims

1. Zinc N-acetyltaurinate of formula:
[CH₃-CO-NH-CH₂-CH₂-SO₃]⁻₂Zn²⁺
for use thereof in the treatment of prostate cancer.
